# EUROPEAN PATENT SPECIFICATION

(11) **EP 1 386 584 B1**
(45) Date of publication and mention of the grant of the patent: **26.09.2012**
(21) Application number: 02717089.3
(22) Date of filing: 10.04.2002
(51) Int. Cl.: G10L 17/00, G06F 19/00, A61B 5/16

(54) **CHAOS-THEORETICAL HUMAN FACTOR EVALUATION APPARATUS**
VORRICHTUNG ZUR CHAOS-THEORETISCHEN HUMANFAKTORBEWERTUNG
APPAREIL D'EVALUATION DE FACTEURS HUMAINS SELON LA THEORIE DU CHAOS

(30) Priority: 16.04.2001 JP 2001116408
(43) Date of publication of application: 04.02.2004
(73) Proprietor: Electronic Navigation Research Institute, an Independent Administrative Institution, Chofu-shi, Tokyo 182-0012 (JP); Mitsubishi Space Software Co., Ltd., Minato-ku, Tokyo 105-6137 (JP); Shiomi, Kakuichi, Minato-ku, Tokyo 105-0021 (JP)
(72) Inventor: SHIOMI, Kakuichi, Minato-ku,, Tokyo 105-0021 (JP); MEGURO, Naritomo, Minato-ku, Tokyo 105-0013 (JP); FURUSE, Nobuhiro, Minato-ku, Tokyo 105-0013 (JP); TANAKA, Hideyuki, Minato-ku, Tokyo 105-0013 (JP)
(74) Representative: Stork Bamberger
(86) International application number: PCT/JP2002/003561
(87) International publication number: WO 2002/085215

(56) References cited:
- JP-A- 2000 113 347
- JP-A- 2000 113 347

## Description

### Technical Field

The present invention relates to a medical diagnosis technology, more specifically to a device for chaological evaluation of human factor for evaluating the condition of the cerebrum by analyzing a human voice and quantitatively detecting a level of the load generated in the human cerebrum. The present invention further relates to a prediction and a determination of changes in the psychosomatic activity of a person during and after his/her examination.

Furthermore, in the present invention, an object to be evaluated is a function of the cerebrum. Since activities of all animals are controlled by the cerebrum, the device of the present invention makes it possible not only to predict and determine the psychosomatic activities, specifically such as a sense of sorrow or pleasure, a sense of dread or uneasiness, those of which tend to surface on the body and can be detected and anticipated by observing or hearing, but also to detect a load arisen on the cerebrum and a psychosomatic condition whilst a person is examined. The aforementioned load is generated in the cerebrum of a person, arising from, for example, an inferiority complex, in solving a complicated mathematical problem, or likes and tastes of a person which relate to feelings of pleasure or discomfort . Also, the psychosomatic activity of a person in the future can be predicted and determined by making a tendency analysis of the person on the basis of the results of the evaluation test. Therefore, the present invention relates widely to an evaluation technology of human factors.

### Background Art

In prior art, the functions of a cerebrum have been obtained by measuring a brain wave. There have been, for example, a method or device for measuring a state of awakening of consciousness such as somnolence or a level of concentration of mind. Since the 1980s, a positron CT equipment (PET) or a single photon CT equipment (SPECT) has so far been used. These types of equipment can offer images of such a moving state of a cerebral blood flow tracer, administered in the brain, or a metabolic rate of glucose in the brain, by using a gamma camera or a dedicated scanner.

Furthermore, equipment exists which measures a state of the brain's activities by detecting the strength of reflected infra-red while irradiating the head with week infra-red and by consecutively inspecting and imaging the activities of a cerebral cortex located inside of the head bone (light topographic equipment).

These conventional methods for measuring brain waves detect an ever-changing electrical change, which is induced by a nerve cell in the cerebral cortex through an electrode attached to a skin of the head. The size of the equipment itself has become comparatively smaller and less expensive. The inspection of the brain wave, however, only gives information about such an abnormal wave caused from paroxysmal disease, a change between sleeping and awakening, a change of an overall level of the brain activity caused by disorder of consciousness, effects on brain functions caused by brain infarction, or brain tumor, or the like.

In an early stage of the study on the brain wave, it was considered that character or mental ability or psychosomatic activities of a person could be determined from the brain wave. But at present it becomes apparent that there is no specific relations between a brain wave and personality or mental ability of a person.

At present, equipment such as PET, SPECT or a light topographic equipment is the highest in sensitivity, being able to analyze and evaluate brain functions, but the size is still large.

Moreover, these types of foregoing equipment are considerably high in price and are difficult to use.

These situations have not been improved since PET, SPECT and the light topographic equipment were put into practical use.

Furthermore, these equipment mentioned above have a sensor to be attached to a human body when in use, or have the necessity of administering an imaging agent to a person to be examined. For example, when performing evaluation of a brain function at a normal state, an attached sensor may cause stress to the person, causing a different psychosomatic activity from that of the person's normal state, thereby information on the brain activity in normal state, which should be used as a standard in evaluation, cannot be obtained, and there is always a possibility that the detection and determination of the psychosomatic condition may not be accurate.

On the other hand, as human activity is completely controlled by the brain, study and grasp on the function of the brain is considered to be a great significance not only in the field of medical technology but also in an area of studies of mental activities of a person engaging in an art such as music or the like and in an area of study on anthropology or civilization that handles human beings as a group with respect to and in connection with a difference in civilization or culture. In order to make use of the evaluation technique of the brain function in the studies of wide areas, it is indispensable to develop a device or equipment capable of evaluating the function of the brain without difficulty.

Therefore, an object of the present invention is to provide a device having a comparatively simple structure and also having an ability to predict and determine a psychosomatic condition of a person by detecting a level of a load generated in the cerebrum, without attaching any parts of the device to the person's body.

### Disclosure of the Invention

In order to solve the above mentioned problems, the inventors of the present invention have been studying strenuously and achieved the invention described hereunder.

The present inventors first conceived that the function of the cerebrum of a person can be evaluated by analyzing a voice uttered by the person. That is, processes in a nerve center of a cerebral cortex when the person utters a word can be classified into two major stages, - - an articulation movement planning stage and a subsequent execution stage of an articulation movement - - . An increase in the number of beats of a word to be uttered or complexity of a word or a sentence to be uttered causes an extension of the processing time in the articulation movement planning stage. These processes cause an increase in the load of the cerebrum, therefore the uttered sound is supposed to vary in line with an increase in the load in the cerebrum.

The uttered sound can be analyzed by a Lyapunov exponent which can be calculated by using a chaological method. A device for judging the current fatigue level and/or a dozing state of a person based on Lyapunov exponents obtained from voice data of the person is known from JP 2000113347A. The inventors have found that the psychosomatic activity can be determined and predicted by measuring a level of a load generated in the cerebrum, without attaching any parts of the device to the body of a person, and by quantitatively expressing the activities of the brain on the basis of the above-mentioned correlation between the cerebrum and the uttered sound.

Consequently, a first aspect of the present invention is that a device for chaological evaluation of human factor comprises the features of claim 1.

According to the first aspect of the present invention, a state of activity of the cerebrum is detected from the state of the Lyapunov exponent calculated by analyzing a digitized sound signal, which is changed into a digital data from a sound uttered by a person, by using a chaological method. The normal state of cerebrum activity can be obtained, because measurement is carried out without giving an unnecessary psychological and physical burden or a feeling of tension to the person to be examined, that is, the measurement is carried out under a non-touching condition of putting no parts of the device such as a sensor or the like on the body of the person to be examined.

In addition, characteristics of changes in the Lyapunov exponent can be detected by chaologically analyzing the uttered sound of a person. Subsequently, relative and temporal changes in the thus obtained Lyapunov exponent can be obtained in real time. A state of the psychosomatic activity of a person such as a state of tension, or tiredness etc. can be easily detected on the basis of the characteristics of the changes in the exponent, by measuring a change in the uttered sound instructed by the cerebrum, more specifically by detecting a state of the load on the cerebrum in an activity of the cerebrum.

Furthermore, the Lyapunov exponents obtained in real time by the chaological analysis and the changes in the obtained real time Lyapunov exponents are always checked relatively and temporally. As the changes in the uttered sound instructed by the cerebrum, - more specifically a state of the load on the cerebrum in the activity of the cerebrum - are measured on the basis of the characteristics of the changes in the exponent, there is no need to prepare in advance a standard value for evaluation. It is possible to make the evaluation immediately during the time of measurement. Furthermore, as there is no need to prepare a standard value for the evaluation beforehand, it is possible to minimize data storage means such as a memory, a magnetic media or the like as much as possible, therefore, it is possible to make the device small, what is more, to realize a built-in type by means of a structure of an on-board type or a chip type.

A second aspect of the present invention is that, in addition to the first aspect of the present invention, the device further comprises: a microphone for inputting the uttered sound of a person as a sound signal; and an analogue-digital conversion means for converting the sound signal, which is input into the microphone, into a digital data.

According to the foregoing aspect of the present invention, when the device comprises the microphone for inputting voices as a sound signal into an uttered sound input means as, what is called, an input signal sensor, and the analogue-digital conversion means for converting the sound signal, which is input into the microphone, into a digital data, it is possible to input voices uttered by a person through an ordinary microphone. Thereby, a person who engages in a work using a microphone such as a pilot of an aircraft, an officer of flight operations, a driver of a public transportation facility, a guide who takes around customers, an announcer of a broadcasting company, a wireless operator or the like, can use a headset or handy microphone as the uttered sound input means.

A third aspect of the present invention is that in addition to one of the first, and second aspects of the present invention, the device further comprises: a non-uttered sound elimination means for eliminating a sound data other than the uttered sound data of a person, on the basis of characteristics of the non-uttered sound data, from said digital data, and transferring the thus obtained non-uttered sound eliminated digital data to said Lyapunov exponent calculation means.

According to the third aspect of the present invention, the sound data other than the uttered sound of a person is eliminated from the digital data of the sound, and the Lyapunov exponent is obtained through the chaological analysis on the basis of the non-uttered sound eliminated digital data. Therefore, it is possible to detect a more accurate state of the load in the activity of the cerebrum because non-uttered sound, what is called a noise, that has no relation to the uttered sound that corresponds to the state of the load in the cerebrum of a human being, is eliminated. For example, in a case where a sound signal such as a signal recorded on a sound communication recorder through wireless at the time of an aircraft accident, or on a voice recorder on an aircraft having a comparatively large amount of noise is handled, investigation on the cause of an accident can be carried out through an analysis in terms of a psychosomatic change or a psychosomatic diagnosis by analyzing the sound signal with the device of the present invention.

Elimination of a sound data other than an uttered sound of a person from a digital data of the sound allows the device to be set up in a place where there is no environmental facility such as a silent room or the like, resulting in making the device more compact and less expensive, even when the device is used as a diagnosis device like a diagnosis device such as PET, SPECT or the like that have been used as medical equipment for diagnosing the state of the activity of a brain or the function of a brain.

A fourth aspect of the present invention is that in addition to one of the first, second, and third aspects of the present invention, the device further comprises an uttered sound detection means for extracting and distinguishing characteristics of an uttered sound of each person from the non-uttered sound eliminated digital data, and transferring the characteristics to said Lyapunov exponent calculation means, enabling to predict and determine the psychosomatic activity of each person in the uttered sounds of more than one person.

According to the foregoing aspect of the present invention, it is possible to analyze and evaluate the uttered sound of each person from the uttered sounds of more than one person because each person's Lyapunov exponent can be obtained respectively by extracting and distinguishing the characteristics of each person's uttered sound from the non-uttered sound eliminated digital data. According to the foregoing aspect, the device can be used for determining a psychosomatic activity of each speaker at a meeting or discussion of more than one person, and for example, a state of mind such as whether or not who got interested in what topic, or whose story, and fear, stress, a state of awakening or the like as well are able to be determined. Therefore, the device may be used as a counseling machine or a lie detector.

The device of the present invention is used for quantifying the activity of various fields of the cerebrum that gives a biological signal as an output from the activity of the cerebrum as a whole, by analyzing the signal through a chaological method.

The present invention enables to evaluate a load generated in the language field of the cerebrum by detecting and evaluating changes of the Lyapunov exponent relatively and temporally through analyzing an uttered sound chaologically and sequentially.

In a chaological analysis, all one needs to do is to pick up an uttered sound signal one-dimensionarily and in time-series, and if the data picked up simultaneously from the same person to be examined are utilized variously, the data are processed separately and finally each evaluation result is combined to give a comprehensive evaluation result.

In addition, each field in the cerebrum is not clearly separated from other fields so that an effect affected by a heavy load in the language field of the cerebrum tends to affect biological signals derived from other fields of the cerebrum.

Furthermore, by measuring the state of a field such as the language field that plays an important roll in a mental activity of a person, the result of the measurement of the language field can be utilized as a scale of the state of the load of the whole cerebrum, if the application of the results is aimed at use for a specific purpose.

### Brief Description of the Drawings

Fig. 1 is a block diagram showing an outline of a personal computer etc. according to an embodiment of the present invention.
Fig. 2 is a block diagram showing an inner structure of the personal computer according to the embodiment of the present invention.
Fig. 3 is a graphical representation showing a relation between time and a Lyapunov exponent of an uttered sound of a person to be examined which was measured by using a device of the present invention.
Fig. 4 is a graphical representation of the present invention that was measured under a different condition other than that of Fig. 3.

### Best Mode for Carrying out the Invention

The present invention will be described hereunder by referring to the accompanying drawings.

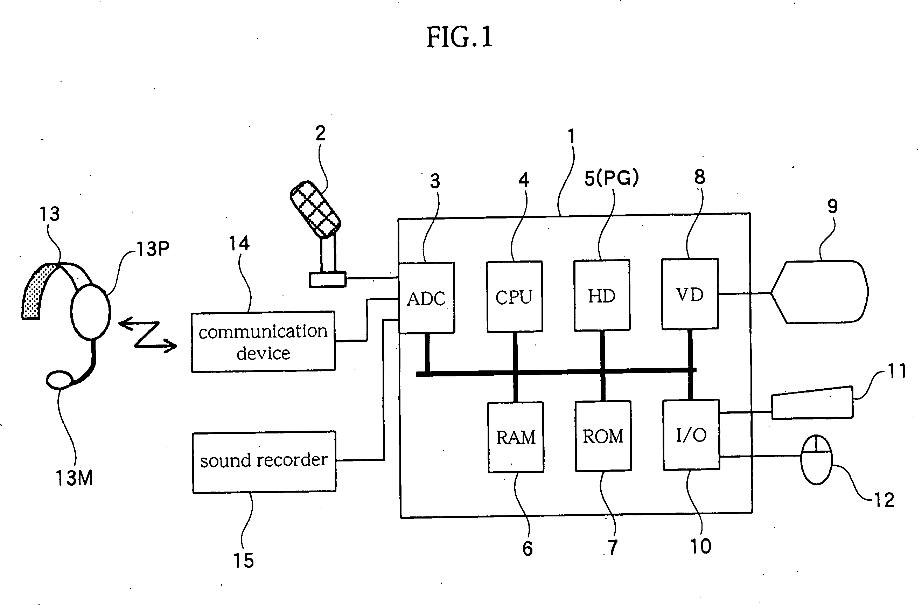

Fig. 1, 2, 3 and 4 show an embodiment of the present invention.

Fig. 1 is a diagram showing an overall configuration in which a device for chaological evaluation of human factor (hereafter referred to as " the device of the present invention") is realized by using a computer.

In Fig. 1, reference numeral 1 shows a computer having a hard disk unit 5 on which a program (hereafter referred to as "cerebrum load detection and determination program") PG is stored to detect and calculate a load on the cerebrum. The hard disk unit 5 also stores each program code of the cerebrum load detection and determination program PG.

It goes without saying that a recording medium such as a flexible disk, CD-ROM, MO or the like and drive units of them, which are usually used for a computer, can also be used.

A chaos analysis program is included in each program code of the cerebrum load detection and determination program PG. The chaos analysis program is a program to calculate the Lyapunov exponent by chaologically analyzing a digital data of a sound read in by the cerebrum load detection and determination program PG.

The computer 1 is connected to a microphone 2, a communication device 14, a sound recorder 15, a video output device 8, and a display unit 9 for displaying an evaluation result and a content of a control command of operation of the device of the present invention.

In addition, an I/O control unit 10 and a keyboard 11 and a mouse 12 which each functions as a key entry means and a pointing device respectively, are connected to the computer 1. Furthermore, a headphone 13P and a head set 13 having a microphone 13M are connected to the communication device 14 via wire or wireless communication.

The microphone 2 or the head set microphone 13M is usually worn by a person to be examined and used in an ordinary working hour of the person to detect and determine a load in the cerebrum. The load in the cerebrum is detected and determined by the device of the present invention. The microphone 2 or the head set microphone 13M is used to pick up, in real time, an uttered sound of the person as a sound signal.

The microphone 2 can be connected and used as well when more than one person simultaneously input their uttered sound.

Further, a sound reproducing unit such as a sound recorder 15, capable of recording a sound signal and reproducing it , can be connected to the computer 1. The sound recorder, for example, like a voice recorder which is usually installed on an airplane and is used to record a sound signal, is used at a time when there is a need to chaologically analyze the recorded sound under certain circumstances, together with the device of the present invention.

The computer 1 comprises CPU 4; RAM 6 and ROM 7 as a storage means for extracting each program code and implementing functions; and a built-in analog-digital converter 3 as "an analog-digital converting means" for taking in a sound signal in the form of a digital data from each device into which sound is input.

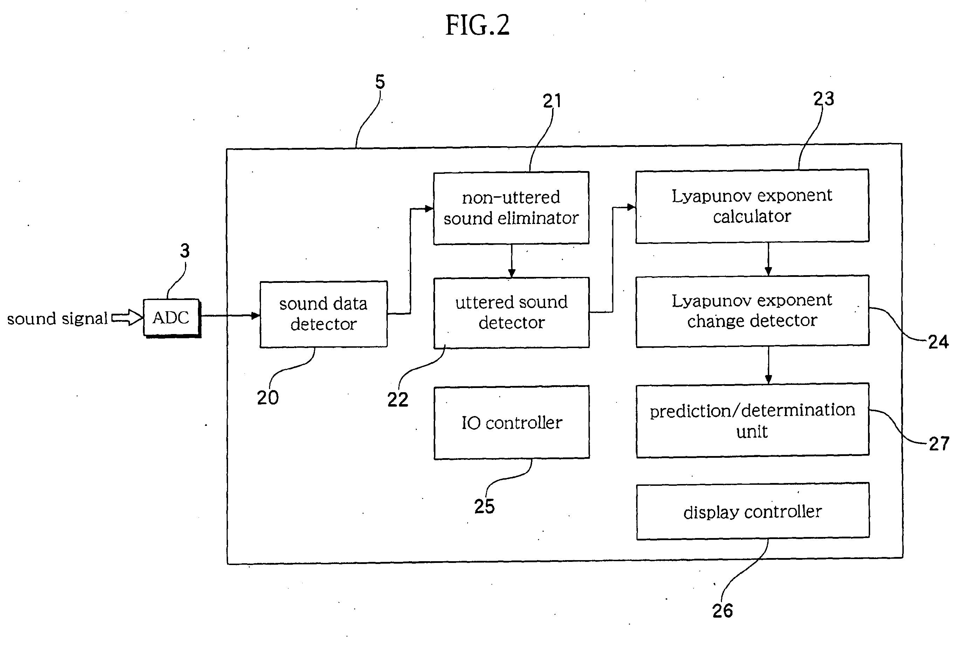

Fig. 2 is a block diagram showing a program stored in a hard disk device 5 of the above mentioned computer 1 of the device of the present invention.

That is, the hard disk device 5 includes a sound data detector 20 into which a signal is sent from the analog-digital converter 3 which is designed to take in a sound signal in the form of a digital data. The sound data detector 20 detects the sound signal from the digital data. The sound data detector 20 inputs a signal into a non-uttered sound eliminator (non-uttered sound elimination means) 21. The non-uttered sound eliminator 21 eliminates a sound other than the uttered sound of a person from the input sound signal.

In addition, a signal is input into the uttered sound detector (uttered sound detection means) 22 from the non-uttered sound eliminator 21 so that the uttered sound is detected from the digital data of a sound by the uttered sound detector 22.
Furthermore, the uttered sound detector 22 sends a signal to a Lyapunov exponent calculator 23 (Lyapunov exponent calculation means) and then the Lyapunov exponent calculator 23 is designed to chaologically analyze and calculate the uttered sound to give the Lyapunov exponent. The Lyapunov exponent calculator 23 sends a signal to a Lyapunov exponent change detector (Lyapunov exponent change detection means) 24, to detect relative and temporal changes in the Lyapunov exponents of the calculated uttered sound with the Lyapunov exponent change detector 24.

Furthermore, a signal from the Lyapunov exponent change detector 24 is input into a prediction/determination unit (prediction/determination means) 27. The prediction/determination means 27 predicts and determines a psychosomatic condition by measuring a level of the load generated in the cerebrum on the basis of a state of the change of the Lyapunov exponent.

In addition, the hard disk device 5 includes IO controller 25 for controlling operation control signals for units and devices, and a display controller 26 for displaying a content of the control command for the operation of the device of the present invention and its evaluation results.

Operation of the device of the present invention will be described hereunder by referring to the block diagram in Fig. 2.

At first the device of the present invention, with which a psychosomatic condition of a person can be evaluated by detecting/determining the level of the load generated in the cerebrum of a person to be examined, takes in an uttered sound of the person to obtain sound signals. More precisely, as shown in Fig. 1, the sound signals can be input through a microphone 13M of a head set 13, for example, when the sound signals are from an aircraft's pilot. The sound signals are sent to the communication device 14 of an airport control tower and then the sound signals are input into the device of the present invention. The sound signals can also be input directly through an ordinary microphone 2 or through the sound recorder 15, that can reproduce the sound signals from a sound-voice recordable media such as an on-board voice recorder on an airplane.

When the input sound signals are analog signals, the analog signals are digitized by the analog-digital converter 3 and then subjected to a data dividing process by using the sound data detector 20 to divide the connected sound signals into a required unit which can be processed in a signal processing procedure which is to be processed later.

Then, the following pre-process is made prior to the execution of chaological analysis. That is, at the non-uttered sound eliminator 21, non-uttered sounds other than the uttered sounds of a person or more than one person are eliminated from the digital data of the sound signals which are obtained at the analog-digital converter 3.

More precisely, the non-uttered sounds to be eliminated are such a sound as a collision damping sound, a collision reverberant sound, a double collision sound, a multiple pile up sound, a crushing sound, a friction sound or the like. These sounds are characterized based on a center frequency, a reverberating time and a band width thereof, then separated and eliminated.

According to the foregoing processes, thus obtained digitized sound data, which does not include the non-uttered sound, is a digital data of the sound only containing the component of the uttered sound of a person. When the obtained digital data of the uttered sound is a mixture of more than one person, each person's uttered sound must be separated from the other's by using the uttered sound detector 22, and then each person's uttered sound is subjected to chaological analysis, separately.

More specifically, all person's uttered sound are characterized separately in advance based on each person's center frequency, a reverberating time and a band width, and then subjected to a comparison procedure with the sound signal measured in real time.

Then, by using the Lyapunov exponent calculator 23, the digitized sound signal of each person's uttered sound is chaologically analyzed to thereby produce the Lyapunov exponent.

More specifically, when uttered sound of a person is processed as a time-series signal, fractal dimension of the waveform thereof has been known to fall on between 5 and 6. The process is executed under a condition that a strange attractor is structured up to 6 dimension. Calculation by the computer can be, for example, performed by a method disclosed in the Laid Open Japan Patent Publication No. 2000-113347 titled "fatigue / drowse detection device and recording media based on voice."

Then, in order to detect and determine the level of the load generated in the cerebrum, the Lyapunov exponent change detector 24 performs a relative comparison between the real time Lyapunov exponent and its standard value, or determines a tendency of a time-series change of the Lyapunov exponent, thereby detecting the amount of change in the level of the load generated in the cerebrum, at the prediction / determination unit 27.

For example, when a person is at work, it is generally said that a certain level of a feeling of tension is required for the person to effectively execute one's work. In prior art, there has been no way of knowing the desirable level of tension at work.

According to the device of the present invention, it is possible to measure the various levels of the load in the cerebrum such as a level at the time in a state where the person is in a relaxed condition, and the level where the person is in a certain level of tension under which the person may become aware of tiredness in a short period of time. Accordingly, it is possible to control the tension of a person at work within a level of moderate tension or an intermediate level of tension, so that the work does not cause serious fatigue to a person during the person's working time.

In the description mentioned above, a person becomes aware of tiredness through a process of gradually accumulating and increasing in the load on the cerebrum when the person is engaging in a routine type of work. On the contrary, when a person is engaging in a creative type of work or work in which a person must act in response to a changeable circumstance, the person becomes aware of tiredness through a continuation of a high level of load on the cerebrum for a specific period of time.

Accordingly, in the present invention, if prediction of a person's psychosomatic condition is intended to be made, it is necessary to apply a prediction algorithm corresponding to the type of work. For example, in a case where a person who engages in work having a tendency of gradual increase in the load of the cerebrum, it is possible to tell him to take a rest under a rule specified in line with a pattern which is obtained through smoothing of temporally changing Lyapunov exponent, by simply using moving average calculation method.

On the contrary, in a case where a person who engages in work which needs to perform a complicated task according to the circumstances, the person's work must be controlled under a rule specified in line with a pattern which is obtained by using Kalman filter or the like or by using a semantic or a knowledge processing like method, instead of a simple arithmetical method.

More specifically, the calculated Lyapunov exponent which changes with time is stored in time-series and temporarily and then a value of the Lyapunov exponent, a change in the Lyapunov exponent and a range of the change are determined in order to quantitatively grasp a change of the state of the cerebrum. As for the method for determining the change between the time-series adjacent ones or the tendency of the change, a moving average calculation method, a least-squares approximation method, a Kalman filter method or the like can be used for producing the characteristic performance curve. Any one of these methods can be chosen.

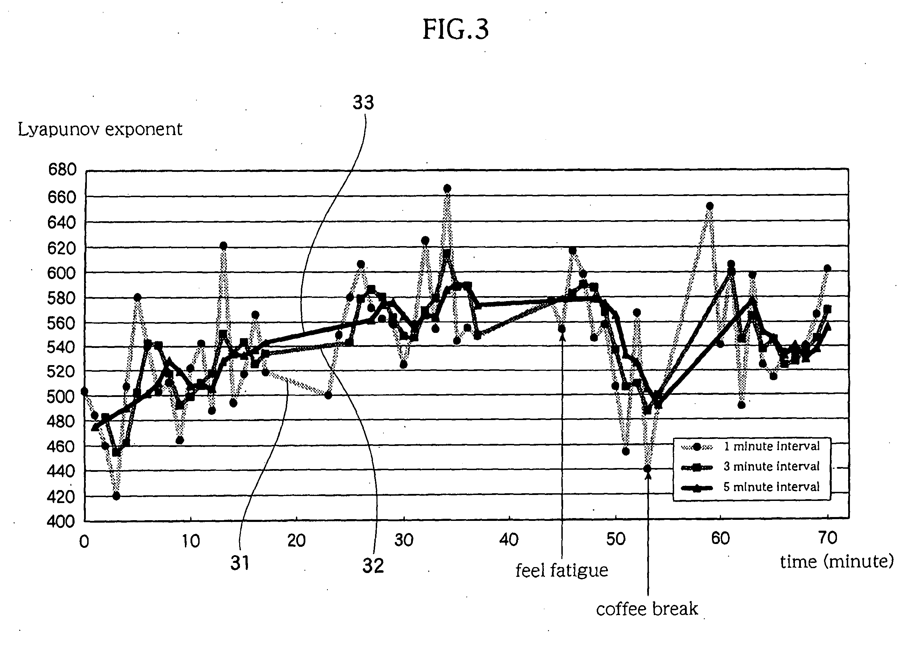

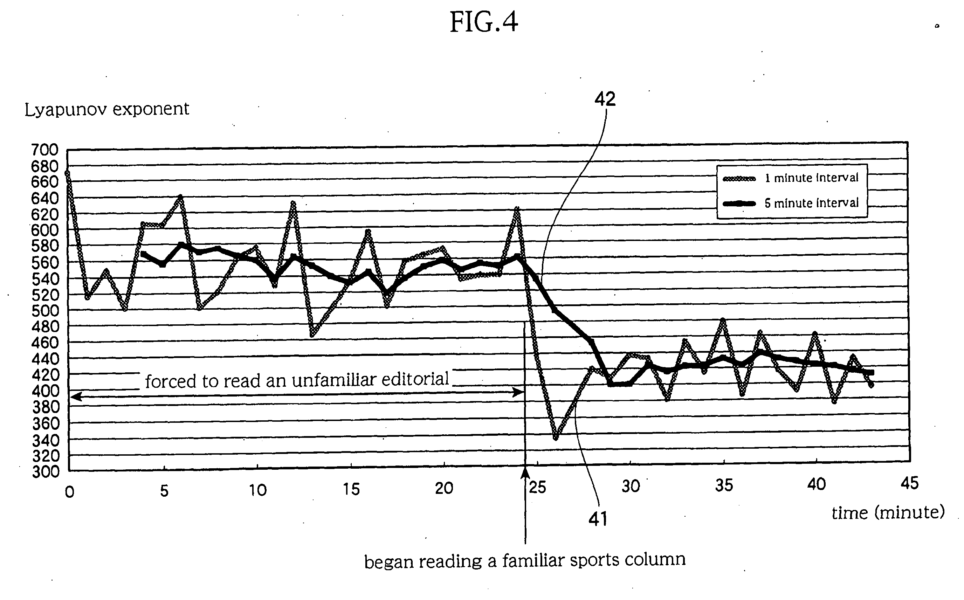

Fig. 3 and Fig. 4 show a time-series graphical representation of the Lyapunov exponent obtained from an uttered sound.

In Fig. 3, a characteristic performance curve 31 shows a curve obtained by calculating moving averages and plotting them at an interval of every one minute. A characteristic performance curve 32 shows a curve obtained by calculating moving averages and plotting them at an interval of every three minutes. A characteristic performance curve 33 shows a curve obtained by calculating moving averages and plotting them at an interval of every five minutes.

As shown in Fig. 4, a characteristic performance curve 41 shows a curve obtained by calculating moving averages and plotting them at an interval of every one minute. A characteristic performance curve 42 shows a curve obtained by calculating moving averages and plotting them at an interval of every five minutes.

As seen from these characteristic performance curves of 31, 32, 33, 41 and 42, a comparatively smoother curve can be obtained when calculated by using a moving average calculation method at an interval of a longer period, so that the tendency of a person's psychosomatic activity can be grasped more clearly.

Then, the aforementioned prediction/determination unit 27 predicts and determines the person's psychosomatic activity or condition by measuring the level of the load generated in the cerebrum on the basis of the characteristic performance curves 31, 32, 33, 41 and 42 which represents a manner of the change in the Lyapunov exponent.

In other words, the temporal change in the Lyapunov exponent shows a level of the load generated in the cerebrum at the moment, that is to say the temporal change of the Lyapunov exponent is just analogous to the measurement of the psychosomatic condition. Accordingly, the measurement of the change of the Lyapunov exponent with time enables us to estimate the tendency of a person. This fact is also obvious from the experimental results. Hereunder, these experimental results will be explained by referring to Fig. 3 and Fig. 4.

Fig. 3 is a time-series data showing a state of increase in the Lyapunov exponent. The data was obtained by calculating the Lyapunov exponent of the strange attractor from the uttered sound of a person assigned to read aloud for more than one hour. Fig. 3 shows that an increase in the Lyapunov exponent was seen in advance before he/she becomes aware of and appeals tiredness.

Increase in the Lyapunov exponent is considered to be a sign of decrease in capacity to adopt to the surrounding environment of the person. Tiredness of the person is said to be a phenomenon as a result of a long time exposure to the state of decrease in the capacity to adopt to the surrounding environment. In other words, when a high level of load is generated in the cerebrum of a person and lasts for a long stretch of time, it results in causing the person to feel tiredness. As mentioned above, in a case where a high level of the Lyapunov exponent continues, it is possible to predict that the person will surely appeal tiredness in due course.

Fig. 4 is a time-series data showing a state of decrease in the Lyapunov exponent. As is the case with the experiment shown in Fig. 3, changes in a person's Lyapunov exponent is plotted in Fig. 4, the data of which was obtained from the person who was reading aloud from a newspaper. After a certain period of time, a sharp decrease in the Lyapunov exponent was observed. This phenomenon can be interpreted as follows. When " the person was forced to read an unfamiliar news item such as an editorial article, there had been a high level of the load generated in the cerebrum. But when the same person moved to read a familiar news item such as a sports column, the level of the load in the person's cerebrum became low."

The device of the present invention enables us to evaluate the human factor of a person by figuring out quantitatively and chaologically the tendency of the person, as mentioned above.

The device of the present invention can be applied to a field of a medical treatment and used as an diagnosing device for a psychosomatic condition of a person. When the device of the present invention is used for such a medical diagnosis area, it is necessary to seek an average value by extracting specific characteristics from many medical examinations. The foregoing requirement can be achieved by further providing the device of the present invention with storage means for storing output results from the uttered sound detector 22 and the Lyapunov exponent change detector 24, and means for performing tendency analysis by using statistical method.

### Industrial Applicability

The present invention can be applied to an area of medical treatment, and also can be applied to a device for chalogical evaluation of a human factor in predicting or determining the state of the activity of the cerebrum, changes in a person's psychosomatic activity at the time of examination, and a tendency of the person's psychosomatic activity in the future after the examination is completed.

## Claims

1. A device for chaological evaluation of human factor comprising:
a Lyapunov exponent calculation means (23) for calculating a Lyapunov exponent by analyzing a digitized sound signal, which is changed into a digital data from a sound uttered by a person, by using a chaological method;
a storage means (6, 7) for storing the calculated Lyapunov exponent which changes over time;
a Lyapunov exponent change detection means (24) for detecting a change in the Lyapunov exponent of the uttered sound, which is calculated by using the Lyapunov exponent calculation means (23); and
an evaluation means ;
**characterized in that**
the Lyapunov exponent change detection means (24) carries out calculation of a moving average calculation method, a least-squares approximation method, or a Kalman filter method using the Lyapunov exponent in order to determine the tendency of the change in the Lyapunov exponent, and
the evaluation means is a prediction and determination means (27) for predicting and determining a psychosomatic activity by measuring a level of a load generated in a cerebrum on the basis of the tendency of the change in the Lyapunov exponent determined by the Lyapunov exponent change detection means (24).

2. A device for chaological evaluation of human factor according to claim 1, the device further comprising a microphone (2, 13M) for inputting the uttered sound of a person as a sound signal and an analogue-digital conversion means (3) for converting the sound signal, which is input into the microphone (2, 13M), into the digital data.

3. A device for chaological evaluation of human factor according to claim 1 or 2, the device further comprising a non-uttered sound elimination means (21) for eliminating a sound data other than the uttered sound data of a person, on the basis of the characteristics of the non-uttered sound, from said digital data, and transferring the non-uttered sound eliminated digital data, to said Lyapunov exponent calculation means (23).

4. A device for chaological evaluation of human factor according to any one of claims 1 to 3, the device further comprising an uttered sound detection means (22) for extracting and distinguishing the characteristics of an uttered sound of each person to be examined from the digital data from the non-uttered sound eliminated digital data, and transferring the characteristics to said Lyapunov exponent calculation means (23), being able to predict and determine the psychosomatic activity of each person from the uttered sounds of more than one person.

## Patentansprüche

1. Einrichtung zur chaostheoretischen Humanfaktorbewertung umfassend:
ein Lyapunov-Exponenten-Berechnungsmittel (23) zum Berechnen eines Lyapunov-Exponenten durch Analysieren eines digitalisierten Lautsignals, das aus einem von einer Person geäußerten Laut in digitale Daten umgewandelt wird, durch Verwenden eines chaostheoretischen Verfahrens;
ein Speichermittel (6, 7) zum Speichern des berechneten Lyapunov-Exponenten, der sich mit der Zeit ändert;
ein Lyapunov-Exponenten-Änderungserkennungsmittel (24) zum Erkennen einer Änderung des Lyapunov-Exponenten des geäußerten Lautes, der unter Verwendung des Lyapunov-Exponenten-Berechnungsmittel (23) berechnet wird; und
ein Bewertungsmittel;
**dadurch gekennzeichnet, dass**
das Lyapunov-Exponenten-Änderungserkennungsmittel (24) unter Verwendung des Lyapunov-Exponenten eine Berechnung eines Verfahrens zum Berechnen eines gleitenden Mittelwerts, eines Approximationsverfahrens der kleinsten Quadrate oder eines Kalman-Filterverfahrens durchführt, um die Tendenz der Änderung des Lyapunov-Exponenten zu bestimmen, und
das Bewertungsmittel ein Vorhersage- und Bestimmungsmittel (27) zum Vorhersagen und Bestimmen einer psychosomatischen Aktivität durch Messen eines Grads einer in einem Großhirn erzeugten Belastung auf Basis der Tendenz der Änderung des Lyapunov-Exponeten ist, die durch das Lyapunov-Exponenten-Änderungserkennungsmittel (24) bestimmt wird.

2. Einrichtung zur chaostheoretischen Humanfaktorbewertung nach Anspruch 1, wobei die Einrichtung weiterhin ein Mikrofon (2, 13M) zum Eingeben des geäußerten Lautes einer Person als ein Lautsignal und ein Analog/Digital-Umwandlungsmittel (3) zum Umwandeln des Lautsignals, das in das Mikrofon (2, 13M) eingegeben wird, in die digitalen Daten umfasst.

3. Einrichtung zur chaostheoretischen Humanfaktorbewertung nach Anspruch 1 oder 2, wobei die Einrichtung weiterhin ein Mittel (21) zum Eliminieren nicht-geäußerter Laute zum Eliminieren von anderen Lautdaten als die geäußerten Lautdaten einer Person auf Basis der Charakteristika der nicht-geäußerten Laute aus den digitalen Daten und Übertragen der nicht-geäußerten eliminierten digitalen Lautdaten an die Lyapunov-Expontenen-Berechnungsmittel umfasst.

4. Einrichtung zur chaostheoretischen Humanfaktorbewertung nach einem der Ansprüche 1 bis 3, wobei die Einrichtung weiterhin ein Mittel (22) zum Erkennen eines geäußerten Lautes zum Extrahieren und Unterscheiden der Charakteristika eines geäußerten Lautes jeder zu prüfenden Person aus den digitalen Daten aus den nicht-geäußerten eliminierten digitalen Lautdaten und Übertragen der Charakteristika an das Lyapunov-Expontenen-Berechnungsmittel (23) umfasst, das in der Lage ist, die psychosomatische Aktivität jeder Person aus den geäußerten Lauten von mehr als einer Person vorherzusagen und zu bestimmen.

## Revendications

1. Dispositif pour l'évaluation chaologique de facteur humain comprenant :
un moyen de calcul d'exposant de Lyapunov (23) pour calculer un exposant de Lyapunov en analysant un signal sonore numérisé, qui est changé en une donnée numérique à partir d'un son prononcé par une personne, en utilisant un procédé chaologique ;
un moyen de stockage (6, 7) pour stocker l'exposant de Lyapunov calculé qui varie dans le temps ;
un moyen de détection de variation d'exposant de Lyapunov (24) pour détecter une variation de l'exposant de Lyapunov du son prononcé, qui est calculé en utilisant le moyen de calcul d'exposant de Lyapunov (23) ; et
un moyen d'évaluation ;
**caractérisé en ce que**
le moyen de détection de variation d'exposant de Lyapunov (24) effectue un calcul d'un procédé de calcul de moyenne mobile, un procédé d'approximation des moindres carrés, ou un procédé par filtre de Kalman utilisant l'exposant de Lyapunov afin de déterminer la tendance de la variation de l'exposant de Lyapunov, et
le moyen d'évaluation est un moyen de prédiction et de détermination (27) pour prédire et déterminer une activité psychosomatique en mesurant un niveau d'une charge générée dans un cerveau en se fondant sur la tendance de la variation de l'exposant de Lyapunov déterminée par le moyen de détection de variation d'exposant de Lyapunov (24).

2. Dispositif pour l'évaluation chaologique de facteur humain selon la revendication 1, le dispositif comprenant en outre un microphone (2, 13M) pour entrer le son prononcé par une personne sous la forme d'un signal sonore et un moyen de conversion analogique-numérique (3) pour convertir le signal sonore, qui est entré dans le microphone (2, 13M), en une donnée numérique.

3. Dispositif pour l'évaluation chaologique de facteur humain selon la revendication 1 ou 2, le dispositif comprenant en outre un moyen d'élimination de son non-prononcé (21) pour éliminer une donnée de son autre que la donnée de son prononcé par une personne, en se fondant sur les caractéristiques du son non-prononcé, à partir de ladite donnée numérique éliminée de son non-prononcé, et pour transférer le son non-prononcé vers ledit moyen de calcul d'exposant de Lyapunov (23).

4. Dispositif pour l'évaluation chaologique de facteur humain selon l'une quelconque des revendications 1 à 3, le dispositif comprenant en outre un moyen de détection de son prononcé (22) pour extraire et distinguer les caractéristiques d'un son prononcé par chaque personne à examiner à partir de la donnée numérique provenant de la donnée numérique éliminée de son non-prononcé, et pour transférer les caractéristiques vers ledit moyen de calcul d'exposant de Lyapunov (23), apte à prédire et déterminer l'activité psychosomatique de chaque personne à partir des sons prononcés de plus d'une personne.
